# EUROPÄISCHE PATENTSCHRIFT

(11) **EP 4 171 718 B1**
(45) Veröffentlichungstag und Bekanntmachung des Hinweises auf die Patenterteilung: **01.07.2026**
(21) Anmeldenummer: 21740435.9
(22) Anmeldetag: 28.06.2021
(51) Int. Cl.: A61M 39/12, A61J 1/14, A61J 1/20, B65D 51/00

(54) **SYSTEM UMFASSEND EINE KAPPE FÜR MEDIZINISCHEN FLUIDBEHÄLTER UND EIN ANSATZTEIL, MEDIZINISCHER FLUIDBEHÄLTER, VERFAHREN ZUM HERSTELLEN EINES FLUIDBEHÄLTERS**
SYSTEM COMPRISING A CAP FOR MEDICAL FLUID CONTAINER AND AN ATTACHMENT, MEDICAL FLUID CONTAINER, METHOD OF MANUFACTURING A FLUID CONTAINER
SYSTEME COMPRENANT UN BOUCHON POUR RECIPIENT DE FLUIDE MEDICAL ET UN ACCESSOIRE, RECIPIENT DE FLUIDE MEDICAL, MÉTHODE DE FABRICATION D'UN RECIPIENT DE FLUIDE

(30) Priorität: 30.06.2020 DE 102020208146
(43) Veröffentlichungstag der Anmeldung: 03.05.2023
(73) Patentinhaber: B. Braun Melsungen AG, 34212 Melsungen (DE)
(72) Erfinder: FUCHS, Jürgen, 34308 Bad Emstal (DE); HARTUNG, Jürgen, 34298 Helsa (DE)
(74) Vertreter: DREISS Patentanwälte PartG mbB
(86) Internationale Anmeldenummer: PCT/EP2021/067723
(87) Internationale Veröffentlichungsnummer: WO 2022/002863

(56) Entgegenhaltungen:
- EP-A2- 1 010 412
- EP-B1- 0 830 874
- WO-A1-2006/005391
- WO-A1-2016/156242
- WO-A1-2019/238773
- DE-A1- 102007 005 407
- DE-A1- 102008 060 864
- DE-B3- 102007 046 951

## Beschreibung

Zu therapeutischen Zwecken werden in der Human- und Veterinärmedizin Infusionen und Transfusionen durchgeführt. Beispielsweise dienen Infusionen der Verabreichung von Flüssigkeiten (z.B. Wirkstofflösungen oder andere flüssige Arzneimittel) in die Blutbahn eines Patienten. Dazu wird die zu verabreichende Flüssigkeit einem Flüssigkeitsbehälter entnommen und fließt durch ein Infusionsbesteck (auch als "Infusionsset" oder "Infusionsgerät" bezeichnet) zum Patientenzugang. Durch den Patientenzugang gelangt die Flüssigkeit in die Blutbahn des Patienten. Bei dem Patientenzugang kann es sich beispielsweise um eine Venenkanüle oder einen Venenkatheter handeln.

Ein Infusions- oder Transfusionsbesteck weist einen Schlauch auf. Häufig aber nicht zwingend weist das Infusions- oder Transfusionsbesteck eine Tropfkammer auf, die mit dem Schlauch verbunden ist, sodass die Flüssigkeit durch die Tropfkammer in den Schlauch fließt. Das Infusions- oder Transfusionsbesteck kann optional weitere Komponenten umfassen, beispielsweise einen Durchflussregler zur Kontrolle der Fließgeschwindigkeit der Flüssigkeit wie etwa eine Rollenklemme.

Wenn eine Tropfkammer vorhanden ist, wird die Tropfkammer über einen Behälteranschluss mit dem Behälter verbunden, sodass die Flüssigkeit aus dem Behälter in die Tropfkammer gelangen kann. Ansonsten ist der Schlauch mit einem entsprechenden Behälteranschluss versehen.

Bei dem Behälteranschluss kann es sich beispielsweise um eine Stechvorrichtung wie etwa einen Hohldorn handeln, mit dem ein Septum des Behälters durchstochen werden kann und der in seinem Inneren einen oder mehrere Fluidkanäle aufweist. Eine derartige Stechvorrichtung wird allgemein als "Spike" bezeichnet. Es sind daneben weitere Systeme bekannt, um das Infusions- oder Transfusionsbesteck mit dem Behälter zu verbinden, beispielsweise Kupplungssysteme mit Steckverbindungen.

Unter einem "Septum" wird ein Behälterverschluss verstanden, der eine Gummimembran oder eine Membran aus einem anderen geeigneten elastischen Material aufweist. Die Membran wird mit einer hohlen Stechvorrichtung durchstochen, um dem Behälter ein darin befindliches Fluid zu entnehmen oder ein Fluid in das Innere des Behälters einzubringen. Bei der Stechvorichtung kann es sich um das oben bereits beschriebene Spike, um eine Kanüle, eine Injektionsnadel etc. handeln. Die Membran ist selbstabdichtend, d.h. wenn die Stechvorrichtung aus der Membran wieder herausgezogen wird, verschließt sich die von der Stechvorrichtung gestochene Öffnung zumindest teilweise, bevorzugt vollständig. Die Membran eines Septums weist demnach keine vorgefertigte Öffnung in Form einer Bohrung oder eines Schlitzes auf, in die die Stechvorrichtung eingeführt wird.

Unter einem "Fluid" wird ein fließfähiges Material verstanden, insbesondere eine Flüssigkeit, ein Gas, eine Suspension oder ein Aerosol.

Ein Behälterzugang wird im Fall von medizinischen Fluidbehältern allgemein als "Port" bezeichnet. Der Port ist demnach der Bereich des Behälters, an den eine externe Vorrichtung zur Fluidentnahme oder Fluidzugabe angeschlossen wird, um eine Fluidverbindung zwischen dem Inneren des Behälters und der externen Vorrichtung herzustellen.

Als "medizinische Fluidbehälter" werden Behälter verstanden, die zur Aufnahme medizinischer Fluide bestimmt sind und aus einem dafür geeigneten Material bestehen. Medizinische Fluide sind beispielsweise flüssige Arzneimittel, flüssige Arzneimittelkomponenten und Blut zu Transfusionszwecken.

Bei dem Flüssigkeitsbehälter, dem die zu verabreichende Flüssigkeit entnommen wird, kann es sich um eine Flasche aus einem starren Material wie beispielsweise Glas handeln. Daneben haben sich am Markt Kunststoffflaschen mit einer gewissen Flexibilität oder Kunststoffbeutel etabliert. Kunststoff hat nicht nur den Vorteil, im Vergleich zu Glas ein geringeres spezifisches Gewicht aufzuweisen und weniger zerbrechlich zu sein. Bei Verwendung eines ausreichend flexiblen Kunststoffs ergibt sich als weiterer Vorteil, dass der Behälter kollabiert, wenn daraus Flüssigkeit entnommen wird. Das heißt, das Volumen des Behälterinneren passt sich dem abnehmenden Volumen der darin befindlichen Flüssigkeit kontinuierlich an sodass keine zusätzlichen Vorkehrungen zum Druckausgleich in Form eines Lufteinströmkanals oder einer Lufteinstömöffnung erforderlich sind, was nicht nur konstruktive Vereinfachungen mit sich bringt sondern auch die Gefahr von Kontaminationen des Behälterinneren durch einströmende Luft vermeidet.

Manche der am Markt erhältlichen Behälter für Infusionsflüssigkeiten ermöglichen ein Zudosieren von weiteren Arzneimitteln (sogenanntes "Pharmacy Admixture"). Das Zudosieren erfolgt beispielsweise durch Zuspritzen über einen weiteren am Behälter vorgesehenen Port, der ebenfalls über ein Septum verfügt. Unter "Zuspritzen" wird das Injizieren mittels einer mit einer Injektionsnadel versehenen Injektionsspritze verstanden, wobei die Injektionsnadel durch das Septum gestochen wird. Üblicherweise ist dieser weitere Port an einer Stelle des Behälters in der Nähe der Position des Ports, über den der Behälteranschluss des Infusions- oder Transfusionsbestecks mit dem Behälter verbunden wird, angeordnet. Dadurch, dass mehrere Ports vorhanden sind, kann gleichzeitig Flüssigkeit entnommen und Flüssigkeit zugeführt werden. Dadurch ist es möglich, während der Durchführung der Infusion ein Arzneimittel zuzudosieren.

Im Stand der Technik bekannt und am Markt erhältlich sind Behälter für Infusionsflüssigkeiten aus einem Polyolefin-Material, die am während der Infusion nach unten weisenden Behälterkopf eine Kappe aufweisen, wobei die Kappe zwei nebeneinander befindliche Ports aufweist. Eine Kappe dieser Art wird auch als "Twinportkappe" bezeichnet. Die beiden Ports sind gleich aufgebaut und erlauben jeweils das Durchstechen eines Septums mit einem Spike und das Zuspritzen mittels einer mit einer Injektionsnadel versehenen Injektionsspritze. Beim Durchstechen eines Septums wird gleichzeitig die unter dem Septum liegende Behälterwandung durchstochen. Die beschriebenen Behälter weisen daher den Vorteil auf, dass sie nicht durch ein Septum abgedichtet werden, sondern dass die Behälterwandung in sich geschlossen ist und den Inhalt des Behälters sicher versiegelt, bevor ein Septum und damit die Behälterwandung erstmals von einer Stechvorrichtung durchstochen worden ist. Das bringt außerdem den produktionstechnischen Vorteil mit sich, dass die Kappe auf den bereits geschlossenen Behälter aufgesetzt wird. Beispielsweise bietet die vorliegende Anmelderin derartige Behälter unter der Handelsbezeichnung Ecoflac^{®} plus an.

Nachteilig am Zuspritzen mittels einer mit einer Injektionsnadel versehenen Injektionsspritze ist die umständliche Handhabung, da eine geeignete Injektionsnadel vorrätig gehalten, ausgepackt, an die Injektionsspritze angeschlossen und anschließend entsorgt werden muss. Nachteilig ist ferner, dass das Durchstechen mit der Injektionsnadel fehleranfällig ist. Insbesondere dann, wenn das Durchstechen nicht vertikal zum Septum erfolgt, kann die Injektionsnadel verbiegen und/oder das Septum dichtet nach dem Herausziehen der Injektionsnadel nicht vollständig ab. Nachteilig ist ferner, dass das Hantieren mit einer Injektionsnadel ein potentielles Verletzungsrisiko mit sich bringt. Die vorgenannten Nachteile gelten umso mehr, wenn das Zuspritzen während der Infusion erfolgen soll, da der Behälter dann kopfüber angeordnet ist und der Port zum Zuspritzen daher schwer zugänglich ist. Nachteilig ist ferner, dass bei der herkömmlichen Vorgehensweise die Injektionsnadel nur durch den Kontakt mit dem Septum im Bereich des Stichs gehalten wird, sodass sie unbeabsichtigt herausgezogen werden oder herausfallen kann. Die genannten Nachteile werden noch weiter verschärft, wenn die Zuspritzprozedur situationsbedingt sehr rasch erfolgen soll.

Aus DE 10 2007 005 407 A1 ist eine Verschlusskappe für ein Behältnis zur Aufnahme von medizinischen Flüssigkeiten bekannt, die über einen Zuspritzteil und einen Entnahmeteil verfügt. Der Entnahmeteil weist eine durchstechbare Membran auf, durch die in herkömmlicher Weise ein Spike eines Infusionsbestecks gestochen werden kann. Der Zuspritzteil ist ausgebildet, den männlichen Konus einer Injektionsspritze aufzunehmen. Der Zuspritzteil weist kein Septum auf, sondern ein Schlitzventil, d.h. eine Membran mit einem Schlitz, der von dem eingeführten männlichen Spritzenkonus geöffnet wird. Die Verschlusskappe der DE 10 2007 005 407 A1 ermöglicht zwar den nadellosen Anschluss einer Injektionsspritze an den Zuspritzteil, jedoch ist es nicht möglich, an den Zuspritzteil eine Stechvorrichtung wie beispielsweise ein Spike anzuschließen. Ferner ist es nicht möglich, die Kappe der DE 10 2007 005 407 A1 auf einen Behälter mit geschlossener Behälterwandung aufzusetzen, weil der Spritzenkonus nicht geeignet wäre, eine unterhalb des Schlitzventils liegende Behälterwandung zu durchstoßen. Das führt dazu, dass ein Behältnis gemäß DE 10 2007 005 407 A1 eine Öffnung aufweist, die nur durch das Schlitzventil gegenüber der Umgebung verschlossen ist. Ein Schlitzventil neigt zu Leckagen, insbesondere, wenn im Behälter ein Überdruck herrscht oder der Behälter kopfüber angeordnet ist. Abgesehen von einem möglichen unerwünschten Flüssigkeitsverlust kann es zum Eintritt von Kontaminationen durch das Schlitzventil kommen. Gemäß DE 10 2007 005 407 A1 ist das Zuspritzteil mit einem Abbrechteil verschlossen. Dieses Abbrechteil ist fertigungstechnisch und in der Handhabung aufwendig. Zudem steht die Funktion des Abbrechteils nicht mehr zur Verfügung, wenn es vom Benutzer bestimmungsgemäß abgebrochen worden ist. In der WO 2006/005391 A1 ist ein steriler Port zum Anschluss einer Schlauchleitung an einen Behälter zur Aufnahme einer enteralen Nährlösung beschrieben. Der Port weist ein in den Behälter einsetzbares und gegenüber dem Behälter abdichtbares Unterteil mit einem Kanal, ein auf dem Unterteil sitzendes Oberteil mit einem Kanal und ein auf dem Oberteil sitzendes Abbrechteil, das den Kanal des Ports verschließt, auf. Der obere Abschnitt des Oberteils ist als Aufnahmestück, in den ein mit der Schlauchleitung verbundener Spike zur Entnahme der Flüssigkeit einsetzbar ist, ausgebildet. Der untere Abschnitt des Oberteils und der obere Abschnitt des Unterteils sind als einschnappend aufeinandergesetzte Verbindungsstücke ausgebildet, wobei das eine Verbindungsstück mindestens einen vorspringenden Ansatz und das andere Verbindungsstück mindestens eine Ausnehmung aufweist. Die Ausnehmung nimmt den Ansatz zur Verdrehsicherung passend auf. Ausgehend von der obengenannten Situation besteht eine Aufgabe der Erfindung darin, ein verbessertes System umfassend eine Kappe für einen medizinischen Fluidbehälter und ein Ansatzteil sowie einen verbesserten medizinischen Fluidbehälter, insbesondere einen verbesserten Behälter für eine Infusionsflüssigkeit, bereitzustellen. Dadurch soll insbesondere das nadelfreie Zudosieren oder Entnehmen von Flüssigkeit aus dem Behälter ermöglicht oder verbessert werden, d.h. das Zudosieren bzw. Entnehmen ohne Verwendung einer separaten Stechvorrichtung.

Diese Aufgabe wird gelöst durch ein System gemäß Anspruch 1, einen medizinischen Fluidbehälter gemäß Anspruch 9 und ein Verfahren zum Herstellen eines Fluidbehälters gemäß Anspruch 15. Vorteilhafte Ausgestaltungen der Erfindung ergeben sich insbesondere aus den Unteransprüchen. Dabei können die in Unteransprüchen und nachfolgender Beschreibung des Gegenstands zu einem unabhängigen Anspruch angeführten Merkmale auch zur vorteilhaften Ausgestaltung des Gegenstands zu einem anderen Anspruch derselben oder einer anderen Anspruchskategorie verwendet werden.

Bei dem erfindungsgemäßen System handelt es sich um ein System umfassend eine Kappe für einen medizinischen Fluidbehälter und ein Ansatzteil. Insbesondere handelt es sich dabei um eine Kappe für einen Behälter für eine medizinische Flüssigkeit. Die Kappe weist mindestens einen ersten Port zum Ausbilden einer Fluidverbindung zwischen dem Fluidbehälter und einer externen Vorrichtung auf. Bei der externen Vorrichtung handelt es sich um eine Vorrichtung zum Entnehmen von Fluid aus dem Fluidbehälter und/oder eine Vorrichtung zum Zuführen von Fluid in den Fluidbehälter. Beispielsweise handelt es sich bei der externen Vorrichtung um eine Injektionsspritze. Der der erste Port weist ein Septum auf. Wie weiter oben bereits erwähnt wurde, weist ein Septum eine selbstabdichtende Membran aus einem geeigneten elastischen Material wie Gummi o.ä. auf. Die Membran wird mit einer hohlen Stechvorrichtung durchstochen, um dem Behälter ein darin befindliches Fluid zu entnehmen oder ein Fluid in das Innere des Behälters einzubringen. Der erste Port weist zusätzlich eine Verbindungsstruktur auf. Das Ansatzteil weist einen Hohldorn und eine mit dem Hohldorn in Fluidverbindung stehende Anschlussstruktur auf. Die Anschlussstruktur ist zum Anschließen einer Vorrichtung zum Entnehmen von Fluid aus dem Fluidbehälter und/oder einer Vorrichtung zum Zuführen von Fluid in den Fluidbehälter ausgebildet. Die Verbindungsstruktur ist ausgebildet, lösbar oder unlösbar mit einer Komplementärverbindungsstruktur des Ansatzteils so verbunden zu werden, dass der Hohldorn des Ansatzteils das Septum durchsticht.

Der erwähnte Port wird dabei auch dann als "erster Port" bezeichnet, wenn die Kappe nur einen einzigen Port aufweist.

Unter einer "lösbaren Verbindung" zwischen zwei Elementen wird eine reversible Verbindung verstanden, d.h. eine Verbindung, die wieder getrennt werden kann, ohne die Elemente dadurch zu zerstören. Beispiele dafür sind Steckverbindungen, Schraubverbindungen und reversibel einrastende Verbindungen. Unter einer "unlösbaren Verbindung" wird umgekehrt eine irreversible Verbindung verstanden. Ein Beispiel dafür ist eine irreversibel einrastende Verbindung.

Dadurch, dass gemäß der vorliegenden Erfindung der erste Port eine Verbindungsstruktur aufweist, ist es möglich, das Ansatzteil mit dem ersten Port zu verbinden, wobei der Hohldorn des Ansatzteils dabei das Septum des ersten Ports durchsticht. Auf diese Weise ist es möglich, eine Fluidverbindung zwischen dem Inneren eines Behälters, an dem die Kappe angebracht ist, und dem Hohldorn herzustellen. Da der Hohldorn mit einer Anschlussstruktur in Fluidverbindung steht, wird auf diese Weise eine Fluidverbindung zwischen dem Inneren des Behälters und der Anschlussstruktur bereitgestellt. Über die Anschlussstruktur kann eine Fluidverbindung zwischen der externen Vorrichtung und dem Inneren des Behälters hergestellt werden, ohne dass dazu das Septum des ersten Ports von einer Stechvorrichtung der externen Vorrichtung durchstochen werden muss. In anderen Worten wird Fluidverbindung zwischen dem Fluidbehälter und einer Vorrichtung zum Entnehmen von Fluid aus dem Fluidbehälter und/oder einer Vorrichtung zum Zuführen von Fluid in den Fluidbehälter indirekt über das Ansatzteil ausgebildet. In diesem Sinn kann das Ansatzteil als Adapter verstanden werden.

Erfindungsgemäß muss eine externe Vorrichtung demnach keine Stechvorrichtung aufweisen, um mit dem Behälter verbunden zu werden. Auf diese Weise wird beispielsweise die Handhabung beim Zuführen einer Flüssigkeit in den Behälter maßgeblich vereinfacht und sicherer gemacht. So muss beispielsweise zum Zuführen einer in einer Injektionsspritze bereitgestellten Flüssigkeit keine separate Injektionsnadel vorrätig gehalten, ausgepackt, an die Injektionsspritze angeschlossen und anschließend entsorgt werden. Ferner werden Handhabungsfehler, die beim Durchstechen mit einer Injektionsnadel auftreten können, z.B. ein nicht vertikal zum Septum erfolgendes Durchstechen, vermieden werden. Außerdem wird das mit dem Hantieren mit einer Injektionsnadel verbundene Verletzungsrisiko vermieden. Die vorgenannten Vorteile der Erfindung kommen in ganz besonderer Weise zum Tragen, wenn das Zuführen während der Infusion erfolgt soll, da der Behälter dann kopfüber angeordnet ist und der Port zum Zuspritzen daher schwer zugänglich ist. Die vorgenannten Vorteile der Erfindung kommen zudem in ganz besonderer Weise zum Tragen, wenn das Zuführen mehrmals erfolgen soll und/oder wenn der Zufuhrvorgang situationsbedingt schnell erfolgen soll. Darüber hinaus ermöglicht die Verbindungstruktur ein sicheres Verbinden mit dem Ansatzteil und damit ein sicheres Verbinden mit der externen Vorrichtung, sodass ein unbeabsichtigtes Herausziehen des Hohldorns bestmöglich vermieden werden kann.

Die erfindungsgemäße Anschlussstruktur ermöglicht jedoch nicht nur das Herstellen einer Fluidverbindung mit einer Vorrichtung zum Zuführen von Flüssigkeit in den Behälter, sondern in gleicher Weise auch die Fluidverbindung mit einer Vorrichtung zum Entnehmen von Fluid aus dem Behälter. Beispielsweise sind Infusionsbestecke bekannt, die anstelle eines Spikes über eine Kupplungsstruktur verfügen, die nicht zum Durchstechen eines Septums ausgebildet ist. Wenn die Kupplungsstruktur eines derartigen Infusionsbestecks und die Anschlussstruktur komplementär zueinander sind, d.h. wenn die Kupplungsstruktur und die Anschlussstruktur miteinander unter Ausbildung einer Fluidverbindung verbunden werden können, kann das Infusionsbesteck an den Behälter angeschlossen werden, ohne dass dazu ein separates Durchstechen eines Septums erforderlich ist. Außerdem ist es möglich, an die Anschlussstruktur einen Schlauch oder ein Rohr mit einer zur Anschlussstruktur komplementären Struktur anzuschließen, um den Inhalt eines Behälters, an dem die Kappe des erfindungsgemäßen Systems angebracht ist, durch den Schlauch oder durch das Rohr teilweise oder vollständig in einen anderen Behälter zu transferieren.

Die Erfindung ermöglicht es ferner, dass beim Durchstechen des Septums des ersten Ports mit dem Hohldorn des Ansatzteils gleichzeitig die Wandung des Behälters, an dem die Kappe des erfindungsgemäßen Systems angebracht ist, in dem unter dem Septum des ersten Ports liegenden Bereich durchstochen wird. Durch die Erfindung ergeben sich daher besonders vorteilhafte Effekte, wenn die Kappe des erfindungsgemäßen Systems gemeinsam mit einem Behälter verwendet wird, dessen Wandung aus einem Kunststoffmaterial besteht und in sich geschlossen ist, sodass der Inhalt des Behälters sicher versiegelt ist.

Da der erste Port der Kappe des erfindungsgemäßen Systems über ein Septum verfügt, ist der Behälter, an dem die Kappe angebracht ist, für Transport und Lagerung steril und besonders sicher verschlossen, bevor das Ansatzteil angebracht wird oder nachdem das Ansatzteil gegebenenfalls wieder entfernt worden ist. Bevorzugt ist das Septum des ersten Ports während Transport und Lagerung des Behälters, an dem die Kappe angebracht ist, durch eine Abdeckung steril abgedeckt. Als Abdeckung eignet sich beispielsweise eine Siegelfolie wie etwa eine Folie aus Metall und/oder Kunststoff. Durch die Abdeckung kann sichergestellt werden, dass das Septum des ersten Ports auch an der vom Behälter abgewandten Seite steril ist, sodass es vor dem Durchstechen mit dem Hohldorn des Ansatzteils nicht in einem gesonderten Schritt desinfiziert werden muss.

Zusätzlich zu den genannten Vorteilen, die sich durch die vorliegende Erfindung ergeben, ermöglicht die Kappe des erfindungsgemäßen Systems, dass statt des Ansatztteils eine externe Vorrichtung direkt an den ersten Port angeschlossen wird. Beispielsweise kann ein Spike eines Infusionsgeräts durch das Septum des ersten Ports gestochen werden. Oder es kann eine Injektionsnadel durch das Septum des ersten Ports gestochen werden. Auf diese Weise wird durch die Erfindung größtmögliche Flexibilität gewährleistet, da ein Behälter, an dem die Kappe des erfindungsgemäßen Systems angeschlossen ist, gemeinsam mit einer Vielzahl von verschiedenen externen Vorrichtungen zur Fluidentnahme oder Fluidzugabe universell einsetzbar ist. Indem das erfindungsgemäße System aber zusätzlich das Ansatzteil umfasst, ermöglicht es einen vielseitigen Einsatz eines Fluidbehälters.

Vorzugsweise wird die Fluidverbindung zwischen dem Inneren des Behälters und der Anschlussstruktur dadurch ermöglicht, dass im Ansatzteil ein durchgehender Fluidkanal von einem distalen Ende des Hohldorns zu einem proximalen Ende der Anschlussstruktur reicht. Auf diese Weise kann die Funktion des Ansatzteils auf konstruktiv einfache und sichere Weise bereitgestellt werden. Weiter vorzugsweise, weil konstruktiv besonders einfach, ist dabei ein einziger Fluidkanal vorgesehen, was besonders zweckmäßig ist, wenn der verwendete Behälter aus einem flexiblen Material besteht, sodass auf einen Druckausgleichskanal verzichtet werden kann.

Die Lagebezeichnungen "distal" und "proximal" sind dabei folgendermaßen definiert: Das distale Ende des Hohldorns ist dessen Spitze. In anderen Worten ist das distale Ende des Hohldorns das Ende, das in das Innere des Behälters weist, wenn der Hohldorn durch das Septum des ersten Ports der an dem Behälter angebrachten Kappe gestochen worden ist. Das proximale Ende der Anschlussstruktur ist dasjenige Ende der Anschlussstruktur, das von der Kappe bzw. vom Behälter weiter beabstandet ist, wenn das Ansatzteil mit der Kappe verbunden ist.

In einer bevorzugten Ausführungsform der Erfindung weist die Verbindungsstruktur der Kappe des erfindungsgemäßen Systems einen Luer-Konus auf, wodurch eine einfach und schnell handhabbare und vielseitig kompatible Verbindungsstruktur zur Verfügung steht. Sogenannte Luer-Verbindungen sind genormte Verbindungen, die für die Anwendung in der Injektions- und Infusionstechnik und verwandten medizinischen Bereichen bestimmt sind. Die Fluidverbindung zwischen den zu verbindenden Elementen wird dabei durch eine kegelförmige Konstruktion der Verbindungsteile, die als "Luer-Konus" bezeichnet wird, erreicht. Dabei wird der Luer-Innenkonus der einen Verbindungsseite auch als "weiblich" bezeichnet, und der Luer-Außenkonus der Gegenseite auch als "männlich". Bei einer Variante wird lediglich der Außenkonus in den Innenkonus gesteckt, ohne dass Strukturen zur Sicherung der Verbindung vorgesehen sind. Diese Variante wird als "Luer-Ansatz", "Luer-Steck" oder "Luer-Slip" bezeichnet. Bei einer weiteren Variante sind der Außenkonus mit einem Innengewinde und der Innenkonus mit einem dazu komplementären Außengewinde zur Verriegelung der Verbindung versehen. Diese Variante wird als "Luer-Lock" bezeichnet. Die Luer-Technik gestattet eine einfache und schnelle Handhabung und garantiert die Kompatibilität zwischen verschiedenen miteinander zu verbindenden Elementen, selbst wenn sie von verschiedenen Herstellern stammen. Die Luer-Technik ist genormt durch ISO 80369-7.

Dabei ist es bevorzugt, dass der Luer-Konus der Verbindungsstruktur ein weiblicher Luer-Konus ist. Bei Verwendung eines weiblichen Luer-Konus in der Verbindungsstruktur der Kappe kann beispielsweise eine sterile Versiegelung der maßgeblichen Oberflächenregionen in besonders einfacher Weise durch eine Folie realisiert werden.

Optional oder alternativ ist es dabei bevorzugt dass die Verbindungsstruktur eine Verbindungsstruktur vom Luer-Lock-Typ ist. Dadurch wird beispielsweise eine sichere Verbindung zwischen der Kappe und dem Ansatzteil ermöglicht.

In einer bevorzugten Ausführungsform der Erfindung weist die Kappe zusätzlich zu dem obenstehend beschriebenen ersten Port einen zweiten Port auf, wobei der zweite Port vom ersten Port getrennt und vorzugsweise neben diesem angeordnet ist und ebenfalls ein selbstabdichtendes Septum aufweist. Der zweite Port kann dabei optional eine Verbindungsstruktur aufweisen, die analog zu der obenstehend beschriebenen Verbindungsstruktur des ersten Ports aufgebaut ist, um ebenfalls mit einem Ansatzteil der obenstehend beschrieben Art verbunden werden zu können.

In weiteren Ausführungsformen der Erfindung weist die Kappe drei oder mehr als drei Ports auf.

Das Ansatzteil des erfindungsgemäßen Systems weist einen Hohldorn und eine mit dem Hohldorn in Fluidverbindung stehende Anschlussstruktur auf. Die Anschlussstruktur ist zum Anschließen einer Vorrichtung zum Entnehmen von Fluid aus dem Fluidbehälter und/oder einer Vorrichtung zum Zuführen von Fluid in den Fluidbehälter ausgebildet.

Das Ansatzteil des erfindungsgemäßen Systems trägt dazu bei, die obenstehend beschriebenen Effekte und Vorteile der vorliegenden Erfindung zu erzielen.

Erfindungsgemäß weist die Anschlussstruktur des Ansatzteils ein Ventil auf. Dadurch ist es beispielsweise möglich, dass der Behälter, an dem die Kappe des erfindungsgemäßen Systems angebracht ist, auch dann dicht abgeschlossen ist, wenn die Kappe mit dem Ansatzteil verbunden ist und daher der Hohldorn des Ansatzteils das Septum des ersten Ports durchsticht und wenn keine Vorrichtung zum Entnehmen von Fluid aus dem Fluidbehälter und/oder zum Zuführen von Fluid in den Fluidbehälter an die Anschlussstruktur angeschlossen ist. Bei dem Ventil handelt es sich weiter bevorzugt um ein konstruktiv besonders einfaches Schlitzventil.

Ein erfindungsgemäßes System umfasst die Kappe und das Ansatzteil.

Bei dem erfindungsgemäßen medizinischen Fluidbehälter handelt es sich um einen medizinischen Fluidbehälter, insbesondere für eine medizinische Flüssigkeit, umfassend einen Hohlkörper und ein erfindungsgemäßes System.

In einer bevorzugten Ausführunsgform des erfindungsgemäßen medizinischen Fluidbehälters ist der Hohlkörper fluiddicht verschlossen. Das heißt die Behälterwandung ist in sich geschlossen und wird nicht durch ein Septum abgedichtet, bevor die Behälterwandung erstmals durch eine Stechvorrichtung durchstochen worden ist. Dadurch ergeben sich in diesem Zusammenhang obenstehend genannten Vorteile in besonders vorteilhafter Weise.

In einer anderen bevorzugten Ausführunsgform des erfindungsgemäßen medizinischen Fluidbehälters ist der Hohlkörper kollabierbar. Dadurch ergeben sich in diesem Zusammenhang obenstehend genannten Vorteile in besonders vorteilhafter Weise.

Bei dem erfindungsgemäßen Verfahren handelt es sich um ein Verfahren zum Herstellen eines Fluidbehälters, insbesondere eines erfindungsgemäßen medizinischen Fluidbehälters. Das erfindungsgemäße Verfahren umfasst die Schritte:
- Bereitstellen eines Hohlkörpers, bevorzugt eines mit Flüssigkeit befüllten Hohlkörpers, mehr bevorzugt eines mit Flüssigkeit befüllten und fluiddicht verschlossenen Hohlkörpers,
- Anformen einer Kappe eines erfindungsgemäßen Systems an einen Bereich des Hohlkörpers durch Spritzgießen.

Das erfindungsgemäße Verfahren umfasst optional als weiteren Schritt das Formen des Septums des ersten Ports durch Spritzgießen. Das erfindungsgemäße Verfahren umfasst optional als weiteren Schritt das Formen des Septums des zweiten Ports durch Spritzgießen.

Das Anformen der Kappe mittels Spritzgießen bietet im Vergleich zu dem bei diesem Verfahrensschritt konkurrierenden Schweißverfahren beispielsweise den Vorteil der bestmöglichen Prozesskonstanz und Prozesskontrolle.

In einer bevorzugten Ausführunsgform des erfindungsgemäßen Verfahrens wird zum Bereitstellen des Hohlkörpers ein mit Flüssigkeit befüllter und fluiddicht verschlossener Hohlkörper durch Herstellen des mit Flüssigkeit befüllten und fluiddicht verschlossenen Hohlkörpers mittels der Blow-Fill-Seal-Technik bereitgestellt. In anderem Worten ist gemäß dieser bevorzugten Ausführunsgform die Herstellung des Hohlkörpers mittels der Blow-Fill-Seal-Technik Teil der Herstellung des Verfahrens zum Herstellen eines Fluidbehälters.

Das Grundkonzept der Blow-Fill-Seal-Technik besteht darin, dass ein Behälter in einem kontinuierlichen Prozess ohne manuellen Eingriff in einem sterilen, geschlossenen Bereich innerhalb einer Maschine geformt, gefüllt und versiegelt wird. Somit ist die Blow-Fill-Seal-Technik zur aseptischen Herstellung von sterilen Darreichungsformen medizinischer Flüssigkeiten geeignet. Die Blow-Fill-Seal-Technik ist mehrstufig: Typischerweise wird zuerst mittels Extrusion ein schlauchförmiger Rohling gebildet. Dieser extrudierte Schlauch wird dann in eine Form eingeschlossen. Nach dem Formen des Behälters durch Aufblasen in der Form wird der Behälter über einen Dorn mit Flüssigkeit befüllt. Anschließend wird der Behälter abgedichtet. Alle Schritte finden in einem sterilen Bereich der Maschine statt.

Die Blow-Fill-Seal-Technik ist eine robuste Methode für die aseptische Zubereitung von sterilen Arzneimitteln.

Weitere Merkmale, Zweckmäßigkeiten und Vorteile der Erfindung werden nachfolgend anhand von exemplarischen Ausführungsbeispielen unter Bezugnahme auf die angeschlossenen Zeichnungsfiguren beschrieben.
- Fig. 1: zeigt eine Schnittdarstellung einer Kappe eines Systems gemäß einem Ausführungsbeispiel der Erfindung.
- Fig. 2: zeigt eine perspektivische Darstellung eines Ansatzteils des Systems gemäß dem Ausführungsbeispiel.
- Fig. 3: zeigt eine Schnittdarstellung einer medizinischen Fluidbehälters gemäß einem weiteren Ausführungsbeispiel der Erfindung mit dem System umfassend die in Fig. 1 dargestellte Kappe und das in Fig. 2 dargestellte Ansatzteil.
- Fig. 4: zeigt eine perspektivische Darstellung des erfindungsgemäßen medizinischen Fluidbehälters gemäß dem Ausführungsbeispiel, an den eine Vorrichtung zur Fluidentnahme oder Fluidzugabe angeschlossen ist.
- Fig. 5: zeigt eine perspektivische Darstellung des erfindungsgemäßen medizinischen Fluidbehälters, an den eine andere Vorrichtung zur Fluidentnahme ohne Verendung des Ansatzteils angeschlossen ist.

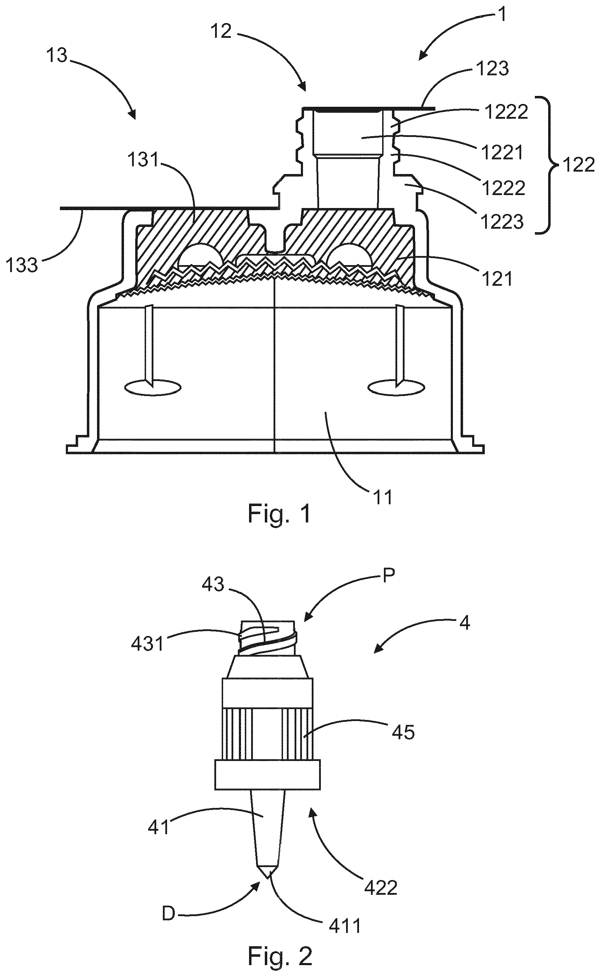

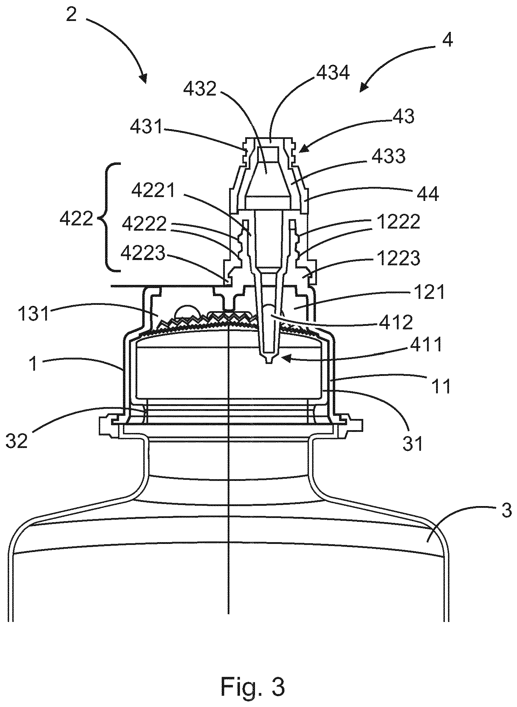

In Fig. 1 ist die Kappe 1 des Systems gemäß einem Ausführungsbeispiel der Erfindung in Schnittdarstellung gezeigt. Weitere Einzelheiten der Kappe 1 werden aus Fig. 3 deutlich, in der der medizinische Fluidbehälter 2 gemäß dem Ausführungsbeispiel der Erfindung. Der medizinische Fluidbehälter 2 umfasst einen Hohlkörper 3, an dem die Kappe 1 angebracht ist. In Fig. 3 ist der medizinische Fluidbehälter 2 inklusive Kappe 1 und Ansatzteil 4 in Schnittdarstellung gezeigt.

Die Kappe 1 weist einen Montagebereich 11 auf, welcher der Anbringung der Kappe 1 am Kopfbereich 31 des Hohlkörpers 3 dient. In dem in den Zeichnungsfiguren dargestellten Ausführungsbeispielen hat der Montagebereich 11 eine helmartige Form, die ausgebildet ist, den Kopfbereich 31 des Hohlkörpers 3 so in sich aufzunehmen, dass die Kappe 1 den Kopfbereich 31 dicht umschließt. Vorzugsweise wird das dadurch erreicht, dass die Kappe 1 durch Spritzgießen an den Kopfbereich 31 angeformt ist, d.h. dass zumindest ein Teil der Struktur der Kappe 1 so durch Spritzgießen geformt wird, dass dieser Teil nach dem Formen fest am Kopfbereich 31 angebracht ist. Beispielsweise kann durch Spritzgießen ein Gehäuse der Kappe 1 (in den Figuren 1 und 3 die Kappe 1 mit Ausnahme der weiter unten beschriebenen Septa 121, 131 und mit Ausnahme der Abdeckungen 123, 133) geformt werden, welches Ausnehmungen aufweist, in die die Septa 121, 131 eingebracht werden.

Die Kappe 1 weist einen ersten Port 12 auf, der zum Ausbilden einer Fluidverbindung zwischen dem Fluidbehälter 2 und einer externen Vorrichtung geeignet ist. Bei der externen Vorrichtung kann es sich um eine Vorrichtung zum Entnehmen von Fluid aus dem Fluidbehälter und/oder eine Vorrichtung zum Zuführen von Fluid in den Fluidbehälter handeln. Beispielsweise handelt es sich bei der externen Vorrichtung um eine Injektionsspritze. Der der erste Port 12 weist ein Septum 121 auf. Das Septum 121 des ersten Ports 12 umfasst ein Elastomer, beispielsweise aus Kautschuk oder synthetischem Kautschuk. Der erste Port 12 weist zusätzlich eine Verbindungsstruktur 122 auf. Die Verbindungsstruktur 122 ist ausgebildet, lösbar oder unlösbar mit einer Komplementärverbindungsstruktur 422 eines Ansatzteils 4, welches einen Hohldorn 41 und eine mit dem Hohldorn 41 in Fluidverbindung stehende Anschlussstruktur 43 aufweist, so verbunden zu werden, dass der Hohldorn 41 das Septum 121 des ersten Ports 12 durchsticht. Das Ansatzteil 4 wird weiter unten näher beschrieben. In dem in den Zeichnungsfiguren dargestellten Ausführungsbeispielen umfasst die Verbindungsstruktur 122 einen Innenkonus 1221, eine Außengewindestruktur 1222 sowie einen umlaufenden Vorsprung 1223 mit hakenförmigen Querschnitt. Der Innenkonus 1221 ist bevorzugt als weiblicher Luer-Konus ausgebildet. Die Außengewindestruktur 1222 kann aus kontinuierlichen Gewindegängen oder aus Gewindenocken bestehen. Der umlaufende Vorsprung 1223 stellt ein Element einer Einrastverbindung bereit. In alternativen, nicht in den Zeichnungsfiguren dargestellten Ausführungsbeispielen, ist die Verbindungsstruktur 122 in anderer Weise gebildet. Beispielsweise kann die Verbindungsstruktur aus einem Innenkonus oder einem Außenkonus alleine bestehen. Zusätzlich kann sie eine Gewindestruktur aufweisen. Alternativ oder zusätzlich kann sie vorspringende und/oder vertiefte Strukturen als Elemente einer Einrastverbindung aufweisen. Einraststrukturen können in anderen Ausführungsbeispielen auch fehlen.

In den in den Zeichnungsfiguren dargestellten Ausführungsbeispielen weist die Kappe 1 einen zweiten Port 13 auf. Der zweite Port 13 weist ein Septum 131 auf. Der zweite Port weist in den in den Zeichnungsfiguren dargestellten Ausführungsbeispielen keine Verbindungsstruktur analog zur Verbindungsstruktur 122 des ersten Ports 12 auf. Über den zweiten Port 13 kann eine weitere Fluidverbindung zwischen dem Fluidbehälter 2 und einer externen Vorrichtung zum Entnehmen von Fluid aus dem Fluidbehälter 2 und/oder zum Zuführen von Fluid in den Fluidbehälter 2 ausgebildet werden. Dazu wird das Septum 131 des zweiten Ports 13 von einer Stechvorrichtung dieser externen Vorrichtung durchstochen.

In weiteren, nicht in den Zeichnungsfiguren dargestellten Ausführungsbeispielen weist der zweite Port 13 eine Verbindungsstruktur analog zur Verbindungsstruktur 122 des ersten Ports 12 auf.

In weiteren, nicht in den Zeichnungsfiguren dargestellten Ausführungsbeispielen ist kein zweiter Port vorgesehen.

In weiteren, nicht in den Zeichnungsfiguren dargestellten Ausführungsbeispielen weist die Kappe 1 zusätzlich zum ersten Port 12 und zum zweiten Port 13 noch mindestens einen weiteren Port auf.

Vorzugsweise ist jeder Port der Kappe 1 durch eine Abdeckung vor Verschmutzungen geschützt. Insbesondere erhält die Abdeckung einen sterilen Zustand der äußeren Oberfläche des Septums. Die Abdeckung wird vorzugsweise entfernt, bevor der Port mit dem Ansatzteil 4 oder mit der Stechvorrichtung externen Vorrichtung verbunden wird. In dem in den Zeichnungsfiguren dargestellten Ausführungsbeispielen handelt es sich bei der Abdeckung jeweils um eine aufgeklebte oder aufgeschweißte Siegelfolie 123, 133, die vom Benutzer vor dem Durchstechen des jeweiligen Septums von Hand abgezogen wird.

Bevor erstmalig der Hohldorn 41 des Ansatzteils 4 oder die Stechvorrichtung einer externen Vorrichtung zur Fluidentnahme bzw. Fluidzugabe in ein Septum 121, 131 eines der Ports 12, 13 gestochen wird, weist der Kopfbereich 31 des Hohlkörpers bevorzugt eine geschlossene Wandung auf, sodass der Hohlkörper 3 selbst ein abgedichteter Hohlkörper ist, der nicht durch die Kappe 1 verschlossen ist.

Bei dem Hohlkörper 3 handelt es sich bevorzugt um einen Hohlkörper aus Kunststoff, mehr bevorzugt aus einem Polyolefin, noch mehr bevorzugt aus Polyethylen, am meisten bevorzugt aus PE-LD.

In den in den Zeichnungsfiguren dargestellten Ausführungsbeispielen weist der Hohlkörper 3 einen Kopfbereich 31 und einen Halsbereich 32 mit einem im Vergleich zum Kopfbereich 31 geringeren Durchmesser auf. Diese Geometrie verbessert das sichere Anbringen der Kappe 1 auf dem Kopfbereich 31. Bevorzugt wird die Kappe 1 an dem Hohlkörper 3 angebracht, indem die Kappe 1 an den Kopfbereich 31 des Hohlkörpers 3 durch Spritzgießen angeformt wird. Der Begriff "Anformen" bedeutet dabei, dass die Kappe 1 nicht vorgefertigt wird, sondern direkt am Kopfbereich 31 des Hohlkörpers 3 ausgebildet wird. Alternativ ist es auch möglich, die Kappe 1 vorzufertigen und am Kopfbereich 31 des Hohlkörpers 3 zu befestigen, beispielsweise durch Schweißen, Kleben oder ein anderes geeignetes Fügeverfahren.

Die Septa 121, 131 können durch Spritzgießen geformt werden. Am meisten bevorzugt ist es, durch Zweikomponenten-Spritzgießen das Gehäuse der Kappe 1 aus einer ersten Materialkomponente und die Septa 121, 131 aus einer zweiten Materialkomponente durch Spritzgießen zu formen.

In Fig. 2 ist das Ansatzteil 4 des Systems gemäß dem Ausführungsbeispiel der Erfindung in perspektivischer Darstellung gezeigt, wobei das Ansatzteil 4 weder mit der Kappe 1 noch mit einer Vorrichtung zum Zuführen bzw. Entnehmen eines Fluids verbunden ist. Die Kappe 1 und das Ansatzteil 4, die in den Figuren 1 und 2 jeweils getrennt dargestellt sind, bilden gemeinsam das System gemäß dem Ausführungsbeispiel. Weitere Einzelheiten des Ansatzteils 4 ergeben sich aus Fig. 3, in der das Ansatzteil 4, das mit der mit einem Hohlkörper 3 verbundenen Kappe 1 verbunden ist, in Schnittdarstellung gezeigt ist.

Das Ansatzteil 4 weist einen Hohldorn 41, eine Komplementärverbindungsstruktur 422 und eine Anschlussstruktur 43 auf.

Der Hohldorn 41 ist ausgebildet, ein Septum zu durchstechen. Der Hohldorn weist an seinem distalen Ende (D) eine Spitze 411 und in seinem Inneren einen Fluidkanal 412 auf. Der Hohldorn 41 und die Komplementärverbindungsstruktur 422 sind so angeordnet, dass der Hohldorn 41 das Septum 121 des ersten Ports 12 durchsticht, wenn das Ansatzteil 4 mit der Kappe 1 verbunden wird, indem die Verbindungsstruktur 122 und die Komplementärverbindungsstruktur 422 miteinander verbunden, d.h. miteinander in Eingriff gebracht werden.

Die Komplementärverbindungsstruktur 422 ist ausgebildet, mit der Verbindungsstruktur 122 der Kappe 1 verbunden zu werden. Daher sind die Verbindungsstruktur 122 und die Komplementärverbindungsstruktur 422 so aufeinander abgestimmt, dass eine lösbare oder unlösbare Verbindung möglich ist, d.h. die Komplementärverbindungsstruktur 422 muss in diesem Sinne zur Verbindungsstruktur 122 komplementär sein, sodass sie miteinander in Eingriff gebracht werden können. In den in den Zeichnungsfiguren dargestellten Ausführungsbeispielen umfasst die Komplementärverbindungsstruktur 422 einen zum Innenkonus 1221 der Verbindungsstruktur 122 komplementären Außenkonus 4221, eine zur Außengewindestruktur 1222 der Verbindungsstruktur 122 komplementäre Innengewindestruktur 4222 sowie eine zum umlaufenden Vorsprung 1223 der Verbindungsstruktur 122 komplementäre Vertiefung 4223 auf. Der Außenkonus 4221 ist bevorzugt als männlicher Luer-Konus ausgebildet. Die Innengewindestruktur 4222 kann dabei aus kontinuierlichen Gewindegängen oder aus Gewindegangabschnitten bestehen. Die umlaufende Vertiefung 4223 stellt gemeinsam mit dem umlaufenden Vorsprung 1223 eine Einrastverbindung bereit. In alternativen, nicht in den Zeichnungsfiguren dargestellten Ausführungsbeispielen ist die Komplementärstruktur 422 entsprechend einer in anderer Weise gebildeten Verbindungsstruktur 122 gebildet. In diesem Zusammenhang wird auf die obenstehende Beschreibung alternativer Ausgestaltungen der Verbindungsstruktur verwiesen.

Die Anschlussstruktur 43 ist zum Anschließen einer externen Vorrichtung zum Entnehmen von Fluid aus dem Fluidbehälter 2 und/oder einer Vorrichtung zum Zuführen von Fluid in den Fluidbehälter 2 ausgebildet.

Dazu weist die Anschlussstruktur 43 in den in den Zeichnungsfiguren dargestellten Ausführungsbeispielen ein Außengewinde 431 und ein Dichtelement 432 auf. Auf das Außengewinde 432 kann ein Innengewinde der externen Vorrichtung zum Entnehmen von Fluid aus dem Fluidbehälter 2 und/oder einer Vorrichtung zum Zuführen von Fluid in den Fluidbehälter 2 aufgeschraubt werden. Das Dichtelement 432 ist ausgebildet, das Ende eines Fluidkanals oder eines Anschlussstutzen der externen Vorrichtung aufzunehmen. Bei dem Anschlussstutzen kann es sich insbesondere um einen männlichen Luer-Konus handeln. Dies ist beispielsweise dann der Fall, wenn als externe Vorrichtung zum Entnehmen von Fluid aus dem Fluidbehälter 2 und/oder einer Vorrichtung zum Zuführen von Fluid in den Fluidbehälter 2 eine Injektionsspritze ohne aufgesetzte Injektionsnadel verwendet wird. Vorliegend ist das Dichtelement 432 als Schlitzventil ausgebildet. Das Schlitzventil umfasst dabei einen Körper 433 aus einem elastischen Material, der in einen Gehäuseabschnitt 44 des Ansatzteils 4 eingepasst ist und der an seinem proximalen Ende einen Schlitz 434 aufweist. Durch den Schlitz 434 kann das Ende des Fluidkanals oder der Anschlussstutzen der externen Vorrichtung eingeführt werden, ohne dass dazu das Dichtelement durchstochen werden muss. Bevor das Ende des Fluidkanals oder der Anschlussstutzen in den Schlitz eingeführt worden ist und nachdem das Ende des Fluidkanals oder der Anschlussstutzen aus dem Schlitz wieder herausgezogen worden ist, ist der Schlitz 434 geschlossen, d.h. die Schnittflächen des Schlitzes 434 dichten gegeneinander ab, sodass das Schlitzventil die Anschlussstruktur 43 fluiddicht abschließt.

Der Gehäuseabschnitt 44 mit der Anschlussstruktur 43 kann beispielsweise auf das übrige Ansatzteil 4 aufgeschweißt oder einstückig mit diesem verbunden sein.

Vorzugsweise erstreckt sich ein durchgehender Fluidkanal durch das Ansatzteil 4, d.h. ein Fluidkanal, der von einem distalen Ende (D) des Hohldorns 41 zu einem proximalen Ende (P) der Anschlussstruktur 43 reicht.

Um das Ansatzteil 4 zum Verbinden mit der Kappe 1 möglichst ergonomisch zu gestalten, kann an dessen Außenfläche eine Griffstruktur 45 beispielsweise in Form von Rillen, Noppen oder einem aufgerauten Bereich vorgesehen sein.

Mit der Bezeichnung "externe Vorrichtung" wird zum Ausdruck gebracht, dass diese Vorrichtung zur Fluidentnahme oder Fluidzugabe nicht als Teil des erfindungsmäßen Systems umfassend die Kappe 1 und das Ansatzteil 4 und nicht als Teil des erfindungsgemäßen medizinischen Fluidbehälters betrachtet wird.

Wenn einerseits die externe Vorrichtung zum Entnehmen von Fluid aus dem Fluidbehälter 2 und/oder zum Zuführen von Fluid in den Fluidbehälter 2 mit dem Ansatzteil 4 verbunden ist und andererseits das Ansatzteil 4 mit dem ersten Port 12 der Kappe 1, die an einem Hohlkörper 3 angebracht ist, verbunden ist, dann wird dadurch eine Fluidverbindung zwischen dem Fluidbehälter 2 und der externen Vorrichtung ausgebildet. Dies geschieht dann in indirekter Weise, d.h. die Fluidverbindung wird dann durch das Ansatzteil 4 vermittelt.

Wenn mit einer Stechvorrichtung einer externen Vorrichtung zum Entnehmen von Fluid aus dem Fluidbehälter 2 und/oder zum Zuführen von Fluid in den Fluidbehälter 2 das Septum 121 des ersten Ports 12 durchstochen wird, dann wird dadurch eine Fluidverbindung zwischen dem Fluidbehälter 2 und der externen Vorrichtung ausgebildet. Dies geschieht dann in direkter Weise, d.h. die Fluidverbindung wird ohne Vermittlung durch das Ansatzteil 4 bereitgestellt.

Im Betrieb wird das Ansatzteil 4 vom Benutzer mit dem ersten Port der Kappe 1 verbunden, indem die Verbindungsstruktur 122 mit der Komplementärverbindungsstruktur 422 in Eingriff gebracht wird, sofern das Ansatzteil 4 und die Kappe 1 noch nicht miteinander verbunden sind. Die Kappe 1 ist dabei an einem Hohlkörper 3 angebracht. Je nach Art der Verbindungsstruktur 122 und der Komplementärverbindungsstruktur 422 wird das Ansatzteil 4 auf die Kappe 1 aufgeschraubt, aufgesteckt oder in sonstiger Weise mit der Kappe 1 verbunden. Beispielsweise wird das Gewinde 4222 des Ansatzteils 4 so lange auf das Gewinde 1222 der Verbindungsstruktur aufgeschraubt, bis elastische Einraststrukturen (beispielsweise in Form des Vorsprungs 1222 und der Vertiefung 4222) ineinander einrasten. Bevorzugt sind die Einraststrukturen so ausgebildet, dass das Einrasten hörbar und/oder fühlbar erfolgt, sodass das Erfolgen des Einrastens in einfacher Weise festgestellt werden kann. Beim Verbinden der Verbindungsstruktur 122 mit der Komplementärverbindungsstruktur 422 durchdringt der Hohldorn 41 mithilfe der Spitze 411 das Septum 121 des ersten Ports 12 sowie die Wandung des Kopfbereichs 31 des Hohlkörpers 3. Zumindest die Spitze 411 des Hohldorns 41 ragt dann in das Innere des Hohlkörpers 3, wodurch das Ansatzteil 4 in Fluidverbindung mit dem Inneren des Hohlkörpers steht und die Anschlussstruktur 43 den Anschluss einer externen Vorrichtung gestattet, um ein Fluid aus dem Hohlkörper 3 zu entnehmen und/oder ein Fluid in das Innere des Hohlkörpers einzubringen.

Es ist möglich, dass der medizinische Fluidbehälter 2 so in Verkehr gebracht wird, dass das Ansatzteil 4 bereits mit der Verbindungsstruktur 122 verbunden ist. Es ist aber auch möglich, dass das Verbinden des Ansatzteils 4 mit der Verbindungsstruktur 122 dem Benutzer überlassen wird.

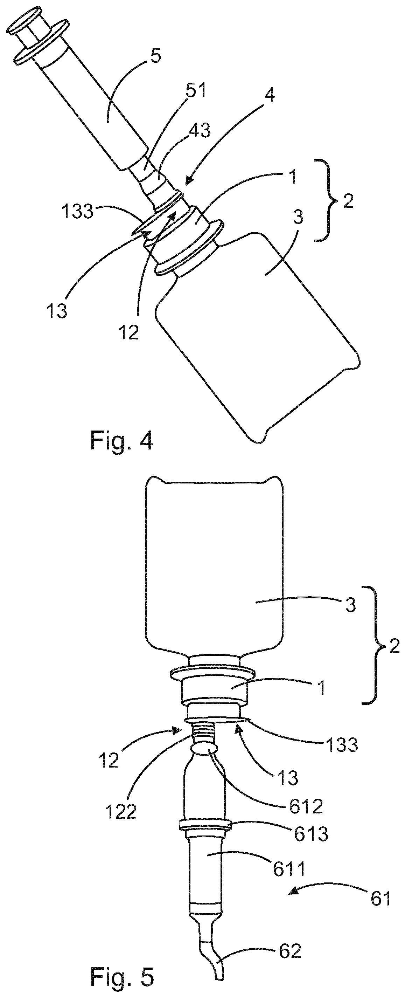

Zum Zuführen einer Flüssigkeit in den medizinischen Fluidbehälter 2 oder zum Entnehmen einer Flüssigkeit aus dem medizinischen Fluidbehälter 2 über den ersten Port 12 kann der Benutzer als externe Vorrichtung zur Fluidentnahme oder Fluidzuführung beispielsweise eine Injektionsspritze 5 mit Luer-Lock-Innengewinde 51 an die Anschlussstruktur 43 des Ansatzteils 4 anschließen, indem er das Luer-Lock-Innengewinde 51 der Injektionsspritze 5 mit dem Außengewinde 431 der Anschlussstruktur 43 verschraubt. Dabei wird der männliche Luer-Konus der Injektionsspritze 5 in eine entsprechende Aufnahme der Anschlussstruktur 43 wie beispielsweise den Schlitz 434 eines Schlitzventils eingeführt. Es besteht dann die gewünschte Fluidverbindung zwischen der Injektionsspritze 5 und dem Inneren des Hohlkörpers 3 des medizinischen Fluidbehälters 2. Diese Situation ist in Fig. 4 dargestellt.

Zusätzlich zu der Möglichkeit eine externe Vorrichtung zum Zufuhren und/oder Entnehmen eines Fluids, im Fall des in Fig. 4 dargestellten Beispiels eine Injektionsspritze 5, an den ersten Port 12 anzuschließen, ohne dabei ein Septum zu durchstechen, besteht erfindungsgemäß zusätzlich die Möglichkeit, eine andere externe Vorrichtung zum Zufuhren und/oder Entnehmen eines Fluids mit einer Stechvorrichtung an den ersten Port 12 anzuschließen, indem mit der Stechvorrichtung das Septum 121 des ersten Ports 12 durchstochen wird. Diese Situation ist in Fig. 5 am Beispiel eines Infusionsbestecks 6 mit einer Tropfkammer 61 und einen daran angeschlossenen Infusionsschlauch 62 dargestellt. Die Tropfkammer 6 verfügt über eine Hohlkammer 611, ein Belüftungsventil 612, einen Griffring 613 und an ihrem in Fig. 5 oberen Ende eine als Hohldorn ausgebildete Stechvorrichtung (in Fig. 5 nicht dargestellt). Mit dem Hohldorn der Tropfkammer 6 wird das Septum 121 des ersten Ports 12 durchstochen, um die gewünschte Fluidverbindung zwischen dem Infusionsbesteck 6 und dem Inneren des Hohlkörpers 3 des medizinischen Fluidbehälters 2 herzustellen. Dabei ist kein Ansatzteil 4 mit der Verbindungsstruktur 122 des ersten Ports 12 verbunden. Das heißt, das erfindungsgemäße System kann in einer Ausführungsform die Möglichkeit bieten, dass der erste Port 12 der Kappe 1 nicht nur gemeinsam mit dem Ansatzteil 4 verwendet werden kann, sondern mit einer geeigneten Vorrichtung zum Entnehmen oder Zuführen von Fluid auch ohne das Ansatzteil 4. Das erfindungsgemäße System gestattet daher einen sehr variablen Einsatz, beispielsweise eines medizinischen Fluzidbehälters für eine Infusionsflüssigkeit oder ein anderes medizinisches Fluid. In diesem Absatz wird die Verbindung des ersten Ports 12 mit einem Infusionsbesteck, welches eine Stechvorrichtung aufwesit, beschrieben, wobei das Ansatzteil nicht zum Einsatz kommt. Ein Infusionsbesteck 6 kann aber auch mit dem Ansatzteil 4, welches mit der Kappe 1 verbunden ist, verbunden werden. Wenn die Verbindung des Infusionsbestecks 6 mit dem Anschlussstruktur 43 des Ansatzteils intendiert ist, wird ein Infusionsbesteck verwendet, das anstelle der Stechvorrichtung oder zusätzlich zu derselben eine Struktur aufweist, die mit der Anschlussstruktur 43 in Eingriff gebracht werden kann.

In den obenstehenden Ausführungsbeispielen des erfindungsgemäßen Systems umfassend die Kappe 1 und das Ansatzteil 4 und des erfindungsgemäßen medizinischen Fluidbehälters 2 ist das Septum 121 des ersten Ports 12 bevorzugt aus einem Material hergestellt, welches ein Silicon umfasst, weiter bevorzugt aus einem Silicon besteht.

Zusätzlich oder alternativ ist in den obenstehenden Ausführungsbeispielen des erfindungsgemäßen Systems umfassend die Kappe 1 und das Ansatzteil 4 sowie des erfindungsgemäßen medizinischen Fluidbehälters 2, in welchen die Kappe 1 einen zweiten Port 13 aufweist, ist das Septum 131 des zweiten Ports 13 bevorzugt aus einem Material hergestellt, welches ein Silicon umfasst, weiter bevorzugt aus einem Silicon besteht.

In den obenstehenden Ausführungsbeispielen des erfindungsgemäßen Systems umfassend die Kappe 1 und das Ansatzteil 4sowie des erfindungsgemäßen medizinischen Fluidbehälters 2 sind alle übrigen Teile der Kappe 1, d.h. alle von einem Septum verschiedenen Teile der Kappe 1, bevorzugt aus einem Material hergestellt, das ein Polyolefin umfasst, weiter bevorzugt aus einem Polyolefin besteht.

In den obenstehenden Ausführungsbeispielen des erfindungsgemäßen Systems umfassend die Kappe 1 und das Ansatzteil 4 sowie des erfindungsgemäßen medizinischen Fluidbehälters ist das Ansatzteil 4 bevorzugt einem Material hergestellt, welches ein Polyolefin umfasst, weiter bevorzugt aus einem Polyolefin besteht.

Entsprechend dieser bevorzugten Wahl der Materialien wird beim erfindungsgemäßen Verfahren zum Herstellen eines Fluidbehälters zum Formen des Septums 121 des ersten Ports 12 bevorzugt ein Material verwendet, welches ein Silicon umfasst, weiter bevorzugt aus einem Silicon besteht.

Entsprechend dieser bevorzugten Wahl der Materialien wird beim erfindungsgemäßen Verfahren zum Herstellen eines Fluidbehälters zum Formen des Septums 131 des zweiten Ports 13 bevorzugt ein Material verwendet, welches ein Silicon umfasst, weiter bevorzugt aus einem Silikon besteht.

Entsprechend dieser bevorzugten Wahl der Materialien wird beim erfindungsgemäßen Verfahren zum Herstellen eines Fluidbehälters zum Formen der übrigen Teile der Kappe 1, d.h. insbesondere zum Anformen der Kappe 1 an einen Bereich 31 des Hohlkörpers 3, bevorzugt ein Material verwendet, welches ein Polyolefin umfasst, weiter bevorzugt aus einem Polyolefin besteht.

## Patentansprüche

1. System umfassend
- eine Kappe (1) für einen medizinischen Fluidbehälter (2), insbesondere für einen Behälter für eine medizinische Flüssigkeit, und
- ein Ansatzteil (4),
wobei die Kappe (1) mindestens einen ersten Port (12) zum Ausbilden einer Fluidverbindung zwischen dem Fluidbehälter (2) und einer Vorrichtung (5, 6) zum Entnehmen von Fluid aus dem Fluidbehälter (2) und/oder einer Vorrichtung (5, 6) zum Zuführen von Fluid in den Fluidbehälter (2) aufweist,
wobei der erste Port (12) ein Septum (121) aufweist,
wobei der erste Port (12) zusätzlich eine Verbindungsstruktur (122) aufweist,
wobei das Ansatzteil (4) einen Hohldorn (41) und eine mit dem Hohldorn (41) in Fluidverbindung stehende Anschlussstruktur (43) aufweist,
wobei die Anschlussstruktur (43) zum Anschließen einer Vorrichtung (5, 6) zum Entnehmen von Fluid aus dem Fluidbehälter (2) und/oder einer Vorrichtung (5, 6) zum Zuführen von Fluid in den Fluidbehälter (2) ausgebildet ist,
wobei der erste Port (12) und das Ansatzteil (4) so ausgebildet sind, dass die Verbindungsstruktur (122) lösbar oder unlösbar mit einer Komplementärverbindungsstruktur (422) des Ansatzteils (4) verbindbar ist und dass der Hohldorn (41) des Ansatzteils (4) das Septum (121) durchsticht, wenn die Verbindungsstruktur (122) und die Komplementärverbindungsstruktur (422) miteinander verbunden werden, und
wobei die Anschlussstruktur (43) ein Ventil (433, 434) aufweist.

2. System gemäß Anspruch 1,
wobei das Ventil ein Schlitzventil ist.

3. System gemäß einem der Ansprüche 1 bis 2,
wobei die Verbindungsstruktur (122) aufweist:
- einen Konus, insbesondere einen Luer-Konus, bevorzugt einen Innenkonus (1221), mehr bevorzugt einen weiblichen Luer-Konus, und/oder
- eine Gewindestruktur (1222) und/oder eine Einraststruktur (1223), die dazu ausgebildet ist, mit einer dazu komplementären Komplementärgewindestruktur (4222) bzw. einer dazu komplementären Komplementäreinraststruktur (4223) des Ansatzteils (4) in Eingriff zu treten oder in Eingriff zu stehen, um die Verbindungsstruktur (122) mit dem Ansatzteil (4) zu verbinden,
wobei am meisten bevorzugt die Verbindungsstruktur eine Verbindungsstruktur vom Luer-Lock-Typ ist.

4. System gemäß einem der Ansprüche 1 bis 3,
wobei die Kappe (1) zusätzlich einen zweiten Port (13) aufweist,
wobei der zweite Port (13) ein Septum (131) aufweist.

5. System gemäß einem der Ansprüche 1 bis 4,
wobei sich ein durchgehender Fluidkanal (412) von einem distalen Ende (D) des Hohldorns (41) zur Anschlussstruktur (43), mehr bevorzugt zu einem proximalen Ende (P) der Anschlussstruktur (43) erstreckt.

6. System gemäß einem der Ansprüche 1 bis 5,
wobei die Anschlussstruktur (43) einen Konus, insbesondere einen Luer-Konus aufweist,
wobei vorzugsweise die Anschlussstruktur einen Innenkonus, insbesondere einen weiblichen Luer-Konus aufweist, und/oder
wobei vorzugsweise die Anschlussstruktur vom Luer-Lock-Typ ist.

7. System gemäß einem der Ansprüche 1 bis 6,
wobei die Anschlussstruktur (43) ein Gewinde (431) aufweist,
wobei vorzugsweise das Gewinde ein Luer-Gewinde ist, und/oder
wobei vorzugsweise das Gewinde ein Außengewinde ist.

8. System gemäß einem der Ansprüche 1 bis 7,
wobei die Komplementärverbindungsstruktur (422) des Ansatzteils (4) eine Komplementärgewindestruktur (4222) aufweist, die zu einer Gewindestruktur (1222) der Verbindungsstruktur (122) der Kappe (1) komplementär ist und dazu ausgebildet ist, mit der Gewindestruktur (1222) der Verbindungsstruktur (122) der Kappe (1) in Eingriff zu treten oder in Eingriff zu stehen, und/oder
wobei die Komplementärverbindungsstruktur (422) des Ansatzteils (4) eine Komplementäreinraststruktur (4223) aufweist, die zu einer Einraststruktur (1223) der Verbindungsstruktur (122) der Kappe (1) komplementär ist und dazu ausgebildet ist, mit der Einraststruktur (1223) der Verbindungsstruktur (122) der Kappe (1) in Eingriff zu treten oder in Eingriff zu stehen.

9. Medizinischer Fluidbehälter (2), insbesondere für eine medizinische Flüssigkeit, umfassend einen Hohlkörper (3) und ein System gemäß einem der Ansprüche 1 bis 8, wobei die Kappe (1) optional mit dem Ansatzteil (4) gekoppelt ist.

10. Medizinischer Fluidbehälter (2) gemäß Anspruch 9,
wobei der Hohlkörper (3) fluiddicht verschlossen ist und/oder
wobei der Hohlkörper (3) kollabierbar ist, und/oder
wobei der Hohlkörper (3) ein Kunststoffhohlkörper, vorzugsweise mit aus Polyethylen gebildeter Wandung, weiter vorzugsweise mit aus PE-LD gebildeter Wandung, ist.

11. Medizinischer Fluidbehälter (2) gemäß einem der Ansprüche 9 bis 10,
wobei der Hohlkörper (3) mit einem Fluid, insbesondere einer medizinischen Flüssigkeit, befüllt ist,
wobei vorzugsweise der mit einem Fluid befüllte Hohlkörper (3) ein Blow-Fill-Seal-Behälter ist.

12. Medizinischer Fluidbehälter (2) gemäß einem der Ansprüche 9 bis 11,
wobei die Kappe (1) durch Spritzgießen an einen Bereich (31) des Hohlkörpers (3) angeformt ist,
wobei vorzugsweise das Septum (121) des ersten Ports (12) und optional das Septum (131) des zweiten Ports (13) durch Spritzgießen an den Bereich des Hohlkörpers angeformt ist bzw. sind,
wobei weiter vorzugsweise die Kappe (1), das Septum (121) des ersten Ports (12) und optional das Septum (131) des zweiten Ports (13) durch Zweikomponentenspritzgießen geformt sind.

13. Medizinischer Fluidbehälter (2) gemäß einem der Ansprüche 9 bis 12,
wobei die Kappe (1) so ausgebildet und so am Hohlkörper (3) angeordnet ist, dass der Hohldorn (41) beim Verbinden des Ansatzteils (4) mit der Verbindungsstruktur (122) das Septum (121) des ersten Ports (12) und einen Bereich einer Wandung des Hohlkörpers (3) durchsticht.

14. Medizinischer Fluidbehälter (2) gemäß einem der Ansprüche 9 bis 13,
wobei der erste Port (12) steril versiegelt ist, vorzugsweise durch eine abziehbare Siegelfolie (123),
wobei optional der zweite Port (13) steril versiegelt ist, vorzugsweise durch eine abziehbare Siegelfolie (133).

15. Verfahren zum Herstellen eines Fluidbehälters, insbesondere eines medizinischen Fluidbehälters (2) gemäß einem der Ansprüche 9 bis 14, umfassend die Schritte:
- Bereitstellen eines Hohlkörpers (3), bevorzugt eines mit Flüssigkeit befüllten Hohlkörpers (3), mehr bevorzugt eines mit Flüssigkeit befüllten und fluiddicht verschlossenen Hohlkörpers (3),
- Bereitstellen eines Systems gemäß einem der Ansprüche 1 bis 8
- Anformen der Kappe (1) an einen Bereich (31) des Hohlkörpers (3) durch Spritzgießen,
- optional Formen des Septums (121) des ersten Ports (12) durch Spritzgießen,
- optional Formen des Septums (131) des zweiten Ports (13) durch Spritzgießen, wobei vorzugsweise zum Bereitstellen des Hohlkörpers (3) ein mit Flüssigkeit befüllter und fluiddicht verschlossener Hohlkörper (3) durch Herstellen des mit Flüssigkeit befüllten und fluiddicht verschlossenen Hohlkörpers (3) mittels der Blow-Fill-Seal-Technik bereitgestellt wird.

## Claims

1. System comprising
- a cap (1) for a medical fluid container (2), in particular for a container for a medical liquid, and
- an attachment part (4),
wherein the cap (1) has at least one first port (12) for forming a fluid connection between the fluid container (2) and a device (5, 6) for removing fluid from the fluid container (2) and/or a device (5, 6) for supplying fluid to the fluid container (2),
wherein the first port (12) has a septum (121),
wherein the first port (12) additionally has a connecting structure (122),
wherein the attachment part (4) has a hollow spike (41) and a connection structure (43) that is in fluid connection with the hollow spike (41),
wherein the connection structure (43) is designed for connecting a device (5, 6) for removing fluid from the fluid container (2) and/or a device (5, 6) for supplying fluid to the fluid container (2),
wherein the first port (12) and the attachment part (4) are designed such that the connecting structure (122) can be detachably or undetachably connected to a complementary connecting structure (422) of the attachment part (4) and such that the hollow spike (41) of the attachment part (4) pierces the septum (121) when the connecting structure (122) and the complementary connecting structure (422) are connected to one another, and
wherein the connection structure (43) has a valve (433, 434).

2. System according to claim 1,
wherein the valve is a slit valve.

3. System according to either of claims 1 to 2,
wherein the connecting structure (122) comprises:
- a cone, in particular a Luer cone, preferably an internal cone (1221), more preferably a female Luer cone, and/or
- a threaded structure (1222) and/or a snap-in structure (1223) that is designed to become engaged with or be engaged with a complementary threaded structure (4222) or a complementary snap-in structure (4223) of the attachment part (4) in order to connect the connecting structure (122) to the attachment part (4),
wherein the connecting structure is most preferably a Luer-Lock type connecting structure.

4. System according to any of claims 1 to 3,
wherein the cap (1) additionally has a second port (13),
wherein the second port (13) has a septum (131).

5. System according to any of claims 1 to 4,
wherein a continuous fluid channel (412) extends from a distal end (D) of the hollow spike (41) to the connection structure (43), more preferably to a proximal end (P) of the connection structure (43).

6. System according to any of claims 1 to 5,
wherein the connection structure (43) has a cone, in particular a Luer cone,
wherein the connection structure preferably has an internal cone, in particular a female Luer cone, and/or
wherein the connection structure is preferably of the Luer-Lock type.

7. System according to any of claims 1 to 6,
wherein the connection structure (43) has a thread (431),
wherein the thread is preferably a Luer thread, and/or
wherein the thread is preferably an external thread.

8. System according to any of claims 1 to 7,
wherein the complementary connecting structure (422) of the attachment part (4) has a complementary threaded structure (4222) which is complementary to a threaded structure (1222) of the connecting structure (122) of the cap (1) and is designed to become engaged with or be engaged with the threaded structure (1222) of the connecting structure (122) of the cap (1), and/or
wherein the complementary connecting structure (422) of the attachment part (4) has a complementary snap-in structure (4223) which is complementary to a snap-in structure (1223) of the connecting structure (122) of the cap (1) and is designed to become engaged with or be engaged with the snap-in structure (1223) of the connecting structure (122) of the cap (1).

9. Medical fluid container (2), in particular for a medical liquid, comprising a hollow body (3) and a system according to any of claims 1 to 8, wherein the cap (1) is optionally coupled to the attachment part (4).

10. Medical fluid container (2) according to claim 9,
wherein the hollow body (3) is sealed in a fluid-tight manner and/or
wherein the hollow body (3) is collapsible, and/or
wherein the hollow body (3) is a plastics hollow body, preferably having a wall made of polyethylene, more preferably having a wall made of PE-LD.

11. Medical fluid container (2) according to either of claims 9 to 10,
wherein the hollow body (3) is filled with a fluid, in particular a medical liquid,
wherein the hollow body (3) filled with a fluid is preferably a blow-fill-seal container.

12. Medical fluid container (2) according to any of claims 9 to 11,
wherein the cap (1) is formed onto a region (31) of the hollow body (3) by injection molding,
wherein the septum (121) of the first port (12) and optionally the septum (131) of the second port (13) is/are preferably formed onto the region of the hollow body by injection molding,
wherein the cap (1), the septum (121) of the first port (12) and optionally the septum (131) of the second port (13) are more preferably formed by two-component injection molding.

13. Medical fluid container (2) according to any of claims 9 to 12,
wherein the cap (1) is designed and arranged on the hollow body (3) such that the hollow spike (41) pierces the septum (121) of the first port (12) and a region of a wall of the hollow body (3) when the attachment part (4) is connected to the connecting structure (122).

14. Medical fluid container (2) according to any of claims 9 to 13,
wherein the first port (12) is sealed so as to be sterile, preferably by means of a removable sealing film (123),
wherein, optionally, the second port (13) is sealed so as to be sterile, preferably by means of a removable sealing film (133).

15. Method for producing a fluid container, in particular a medical fluid container (2) according to any of claims 9 to 14, comprising the steps of:
- providing a hollow body (3), preferably a hollow body (3) filled with liquid, more preferably a hollow body (3) filled with liquid and sealed in a fluid-tight manner,
- providing a system according to any of claims 1 to 8
- forming the cap (1) onto a region (31) of the hollow body (3) by injection molding,
- optionally forming the septum (121) of the first port (12) by injection molding,
- optionally forming the septum (131) of the second port (13) by injection molding, wherein, to provide the hollow body (3), a hollow body (3) filled with liquid and sealed in a fluid-tight manner is preferably provided by producing the hollow body (3) filled with liquid and sealed in a fluid-tight manner by means of the blow-fill-seal technique.

## Revendications

1. Système comprenant
- un capuchon (1) pour un récipient pour fluide médical (2), en particulier pour un récipient pour un liquide médical, et
- une partie formant embout (4),
dans lequel le capuchon (1) comprend au moins un premier orifice (12) permettant de former une liaison fluidique entre le récipient pour fluide (2) et un dispositif (5, 6) permettant de prélever du fluide du récipient pour fluide (2) et/ou un dispositif (5, 6) permettant d'amener du fluide dans le récipient pour fluide (2),
dans lequel le premier orifice (12) présente une cloison (121),
dans lequel le premier orifice (12) présente en outre une structure de liaison (122),
dans lequel la partie formant embout (4) présente un mandrin creux (41) et une structure de raccordement (43) en liaison fluidique avec le mandrin creux (41),
dans lequel la structure de raccordement (43) est conçue pour le raccord d'un dispositif (5, 6) permettant de prélever du fluide du récipient pour fluide (2) et/ou d'un dispositif (5, 6) permettant d'amener du fluide dans le récipient pour fluide (2),
dans lequel le premier orifice (12) et la partie formant embout (4) sont formés de telle sorte que la structure de liaison (122) peut être reliée de manière amovible ou non amovible à une structure de liaison complémentaire (422) de la partie formant embout (4) et que le mandrin creux (41) de la partie formant embout (4) perce la cloison (121) lorsque la structure de liaison (122) et la structure de liaison complémentaire (422) sont reliées l'une à l'autre, et
dans lequel la structure de liaison (43) présente une vanne (433, 434).

2. Système selon la revendication 1,
dans lequel la vanne est une vanne à fente.

3. Système selon l'une des revendications 1 à 2,
dans lequel la structure de liaison (122) présente :
- un cône, en particulier un cône Luer, de préférence un cône interne (1221), plus préférablement un cône Luer femelle, et/ou
- une structure filetée (1222) et/ou une structure d'encliquetage (1223) qui sont formées pour venir en prise ou être en prise avec une structure filetée complémentaire (4222) ou une structure d'encliquetage complémentaire (4223) de la partie formant embout (4), afin de relier la structure de liaison (122) à la partie formant embout (4),
dans lequel, de manière la plus préférée, la structure de liaison est une structure de liaison de type Luer-Lock.

4. Système selon l'une des revendications 1 à 3,
dans lequel le capuchon (1) présente en outre un second orifice (13),
dans lequel le second orifice (13) présente une cloison (131).

5. Système selon l'une des revendications 1 à 4,
dans lequel un canal pour fluide continu (412) s'étend depuis une extrémité distale (D) du mandrin creux (41) jusqu'à la structure de raccordement (43), plus préférablement jusqu'à une extrémité proximale (P) de la structure de raccordement (43).

6. Système selon l'une des revendications 1 à 5,
dans lequel la structure de raccordement (43) présente un cône, en particulier un cône Luer,
dans lequel, de préférence, la structure de raccordement présente un cône interne, en particulier un cône Luer femelle, et/ou
dans lequel, de préférence, la structure de raccordement est du type Luer-Lock.

7. Système selon l'une des revendications 1 à 6,
dans lequel la structure de raccordement (43) présente un filetage (431),
dans lequel, de préférence, le filetage est un filetage Luer, et/ou
dans lequel, de préférence, le filetage est un filetage extérieur.

8. Système selon l'une des revendications 1 à 7,
dans lequel la structure de liaison complémentaire (422) de la partie formant embout (4) présente une structure filetée complémentaire (4222) qui est complémentaire d'une structure filetée (1222) de la structure de liaison (122) du capuchon (1) et est formée pour venir en prise ou être en prise avec la structure filetée (1222) de la structure de liaison (122) du capuchon (1), et/ou
dans lequel la structure de liaison complémentaire (422) de la partie formant embout (4) comprend une structure d'encliquetage complémentaire (4223) qui est complémentaire d'une structure d'encliquetage (1223) de la structure de liaison (122) du capuchon (1) et est formée pour venir en prise ou être en prise avec la structure d'encliquetage (1223) de la structure de liaison (122) du capuchon (1).

9. Récipient pour fluide médical (2), en particulier pour un liquide médical, comprenant un corps creux (3) et un système selon l'une des revendications 1 à 8, dans lequel le capuchon (1) est éventuellement accouplé à la partie formant embout (4).

10. Récipient pour fluide médical (2) selon la revendication 9,
dans lequel le corps creux (3) est fermé de manière étanche aux fluides et/ou
dans lequel le corps creux (3) peut se replier, et/ou
dans lequel le corps creux (3) est un corps creux en matière plastique, de préférence comportant une paroi formée à partir de polyéthylène, plus préférablement comportant une paroi formée à partir de PE-LD.

11. Récipient pour fluide médical (2) selon l'une des revendications 9 à 10,
dans lequel le corps creux (3) est rempli d'un fluide, en particulier d'un liquide médical,
dans lequel, de préférence, le corps creux (3) rempli d'un fluide est un récipient de type formage-remplissage-scellage.

12. Récipient pour fluide médical (2) selon l'une des revendications 9 à 11,
dans lequel le capuchon (1) est moulé par moulage par injection sur une zone (31) du corps creux (3),
dans lequel, de préférence, la cloison (121) du premier orifice (12) et, éventuellement, la cloison (131) du second orifice (13) sont moulées par moulage par injection sur la zone du corps creux,
dans lequel, en outre, de préférence, le capuchon (1), la cloison (121) du premier orifice (12) et, éventuellement, la cloison (131) du second orifice (13) sont moulés par moulage par injection à deux composants.

13. Récipient pour fluide médical (2) selon l'une des revendications 9 à 12,
dans lequel le capuchon (1) est formé et disposé sur le corps creux (3) de telle sorte que le mandrin creux (41) perce la cloison (121) du premier orifice (12) et une zone d'une paroi du corps creux (3) lors de la liaison de la partie formant embout (4) à la structure de liaison (122).

14. Récipient pour fluide médical (2) selon l'une des revendications 9 à 13,
dans lequel le premier orifice (12) est scellé de manière stérile, de préférence par une feuille de scellement pelable (123),
dans lequel, éventuellement, le second orifice (13) est scellé de manière stérile, de préférence par une feuille de scellement pelable (133).

15. Procédé pour la fabrication d'un récipient pour fluide, en particulier d'un récipient pour fluide médical (2) selon l'une des revendications 9 à 14, comprenant les étapes consistant à :
- fournir un corps creux (3), de préférence un corps creux (3) rempli de liquide, plus préférablement un corps creux (3) rempli de liquide et fermé de manière étanche aux fluides,
- fournir un système selon l'une des revendications 1 à 8,
- mouler le capuchon (1) sur une zone (31) du corps creux (3) par moulage par injection,
- éventuellement, mouler la cloison (121) du premier orifice (12) par moulage par injection,
- éventuellement, mouler la cloison (131) du second orifice (13) par moulage par injection, dans lequel, de préférence, pour fournir le corps creux (3), un corps creux (3) rempli de liquide et fermé de manière étanche aux fluides est fourni en fabriquant le corps creux (3) rempli de liquide et fermé de manière étanche aux fluides au moyen de la technique formage-remplissage-scellage.
